# EUROPEAN PATENT APPLICATION

(11) **EP 1 604 658 A2**
(43) Date of publication of application: **14.12.2005**
(21) Application number: 05020308.2
(22) Date of filing: 10.04.2002
(51) Int. Cl.: A61K 31/19, A61K 31/54, A61P 25/06, A61K 45/06, A61K 31/5415

(54) **Pharmaceutical composition comprising ibuprofen and prochlorperazine**

(30) Priority: 10.04.2001 GB 0108930
(62) Divisional of application: 02708550.5
(71) Applicant: The Boots Company PLC, Nottingham, Nottinghamshire NG2 3AA (GB)
(72) Inventor: Pankhania, Mahendra Govind, Nottingham, NG2 3AA (GB); Humphrey, Stephen Philip, Nottingham NG2 3AA (GB)
(74) Representative: Frith, Richard William

(57) **Abstract**

A pharmaceutical composition for the treatment of migraine or overindulgence comprising a therapeutically effective amount of racemic ibuprofen or a pharmaceutically acceptable salt or an enantiomer or a pharmaceutically acceptable salt thereof and a therapeutically effective amount of prochlorperazine or a pharmaceutically acceptable salt thereof.

## Description

This invention relates to pharmaceutical compositions comprising ibuprofen, and in particular to compositions containing ibuprofen and prochlorperazine useful for the treatment of migraine and in the treatment of the nausea and headache resulting from overindulgence.

Ibuprofen, namely 2-(4-isobutylphenyl)propionic acid, is a well known medicament with analgesic, anti-inflammatory and anti-pyretic properties. It is usually sold in the form of racemic ibuprofen (equal amounts of the S(+)-ibuprofen and R(-)-ibuprofen enantiomers). It may also be in the form of the purified form of either enantiomer, especially S(+)-ibuprofen which is acknowledged to be the active form of racemic ibuprofen. Ibuprofen is also available in salt form, for example the sodium or lysine salt of ibuprofen. Ibuprofen is available under prescription (eg Brufen (RTM)), primarily for the treatment of painful and anti-inflammatory disorders including rheumatoid arthritis, ankylosing spondylitis, osteoarthritis, postoperative pain, post partum pain and soft tissue injuries, generally at doses up to 3200 mg per day. Ibuprofen is also available as a non-prescription drug (eg Nurofen (RTM)), primarily for the treatment of symptoms of pain and fever including headache, migraine, rheumatic pain, muscular pain, backache, neuralgia, dysmenorrhoea, dental pain and colds and flu, generally at doses up to 1200mg per day. The commercially available ibuprofen tablets usually contain 200mg, 400 mg, 600 mg or 800 mg racemic ibuprofen. When an enantiomer or salt of ibuprofen is used the amount of active substance present may be such that the same therapeutic effect is obtained as from the presently-available doses of racemic ibuprofen. Hereinafter the term "ibuprofen" means any enantiomer of ibuprofen or mixtures of enantiomers including the racemic mixture.

Prochlorperazine, namely 2-chloro-10-[3-(4-methyl-1-piperazinyl)propyl]-10H-phenothiazine is a well known medicament with antiemetic properties. Pharmaceutically acceptable salts, eg the maleate or mesylate salt of prochlorperazine may be used instead of prochlorperazine itself.

Prochlorperazine is available under prescription for the treatment of nausea, vomiting and vertigo in the form of tablets or suppositories containing the maleate salt of prochlorperazine or in the form of syrups or injection liquids containing the mesylate salt of prochorperazine.

The present inventors have surprisingly found that when a composition comprising ibuprofen and prochlorperazine or their salts is administered to patients experiencing the symptoms of migraine or overindulgence advantageous relief from those symptoms is achieved.

Without wishing to be bound by theory, it is thought that the presence of prochlorperazine may increase the rate of absorption of ibuprofen by the body.

The present invention provides a pharmaceutical composition for the treatment of migraine or overindulgence comprising a therapeutically effective amount of racemic ibuprofen or a pharmaceutically acceptable salt or an enantiomer or a salt thereof and a therapeutically effective amount of prochlorperazine or a pharmaceutically acceptable salt thereof. The compositions of the invention typically also comprise a pharmaceutically acceptable diluent or carrier.

The present invention also provides a method of treating migraine or overindulgence which comprises the administration to a patient in need thereof of a pharmaceutical composition comprising a therapeutically effective amount of racemic ibuprofen or a pharmaceutically acceptable salt thereof, an enantiomer or salt thereof and a therapeutically effective amount of prochlorperazine or a pharmaceutically acceptable salt thereof.

Typically, a suitable amount of racemic ibuprofen to be administered per day is in the range of from 200 to 3200 mg per day in one or more doses to be administered throughout the day. Preferably each dose contains 50 to 800 mg (preferably 50 to 400, more preferably 200 to 400 mg) of racemic ibuprofen or a corresponding amount of or a pharmaceutically acceptable salt thereof, an enantiomer or pharmaceutically acceptable salt thereof. Particularly preferred amounts of racemic ibuprofen per unit dose are 200 and 400 mg.

The amount of prochlorperazine to be administered per day is generally in the range 1 to 30 (preferably 3 to 20) mg per day in one or more doses throughout the day. Preferably each dose contains 1 to 10 mg (preferably 2 to 5 mg, for example 3 or 4 mg) of prochlorperazine. Alternatively, a corresponding amount of a pharmaceutically acceptable salt of prochlorperazine may be used.

By the term "corresponding amount" we mean that if a pharmaceutically acceptable salt of either of the active drugs (eg the sodium or lysine salt of ibuprofen and/or the maleate or mesylate salt of prochlorperazine) is used then the amount of drug to be administered should be such that the same therapeutic effect is achieved as from the doses given above. If an enantiomer of ibuprofen (eg S (+) - ibuprofen) is used, the amount of drug should be such that the same therapeutic effect is achieved as from the doses of racemic ibuprofen given above.

Preferred salts include the sodium or lysine salt of ibuprofen and/or the maleate or mesylate salt of prochlorperazine.

In particularly preferred embodiments, the racemic ibuprofen lysine salt is used. This is because the racemic ibuprofen lysine salt is very quickly absorbed by the body, and significantly faster than racemic ibuprofen in its acid form.

Unlike other anti-emetics, prochlorperazine does not have to reach the lower gut before absorption by the body; it can be absorbed from the buccal cavity onwards. Therefore, a combination of the lysine salt of racemic ibuprofen with prochlorperazine provides a rapid acting composition.

In the following description of suitable dosage forms containing both active drugs the term "active combination" means a combination of ibuprofen (a racemic mixture or a single enantiomer) or a pharmaceutically acceptable salt thereof and prochlorperazine or a pharmaceutically acceptable salt thereof. The active combination may comprise the two drugs in intimate admixture but may also comprise combinations in which the two drugs are kept apart for example by coating one or both drugs or physically separating the two drugs for example by using a bilayer tablet in which one drug is present in each layer. The active combination may be prepared by suitable mixing prior to adding to other components of the desired dosage forms or it may be prepared in situ during the production of the desired dosage forms by adding the two drugs separately to the other components of the dosage forms. The amounts of each drug in the active combination in each dosage form should be such that the desired total dose of each drug is taken when the patient takes the pharmaceutical compositions of the present invention as directed. Suitably the pharmaceutical compositions are administered in divided doses throughout the day so the amount of ibuprofen (or the corresponding amount of a salt thereof) to be administered at each dosing time is in the range 50 to 800 mg (preferably 50 to 400, more preferably 200 to 400) mg and the amount of prochlorperazine (or the corresponding amount of a salt thereof) to be administered at each dosing time is 1 to 10 (preferably 2 to 5) mg. Therefore, if two dosage forms are to be administered at each time the dosage forms should contain 25 to 400 (preferably 25 to 200, more preferably 100 to 200) mg ibuprofen and 0.5 to 5 (preferably 1 to 2.5) mg of prochlorperazine (or the corresponding amounts of their salts).

The pharmaceutical compositions of the present invention may be administered orally, rectally, parenterally, bucally or topically, preferably orally. Suitably the pharmaceutical compositions of the present invention may take the form of any of the known pharmaceutical compositions for oral, rectal, parenteral, buccal or topical administration. Preferably, the compositions of the invention are in a form suitable for oral administration or in the form of a suppository.

Pharmaceutically acceptable diluents or carriers suitable for use in such compositions are well known in the art of pharmacy. The compositions of the invention typically contain 1-99% by weight of active combination.

Solid compositions for oral administration are preferred compositions of the invention. Solid compositions of the invention are preferably prepared in unit dosage form and are the known pharmaceutical forms for such administration, for example tablets and capsules. Suitably tablets may be prepared by mixing the active combination with an inert diluent such as calcium phosphate in the presence of disintegrating agents, for example maize starch, and lubricating agents, for example magnesium stearate, and tableting the mixture by known methods. Such tablets may, if desired, be provided with enteric coatings by known methods, for example by the use of cellulose acetate phthalate. Similarly, capsules, for example hard or soft gelatin capsules, containing the active combination optionally in the form of beads with or without added excipients, may be prepared by conventional means and, if desired, provided with enteric coatings in a known manner. The tablets may be formulated in a manner known to those skilled in the art so as to give a controlled release of the compound of the present invention. Other compositions for oral administration include oily suspensions containing a compound of the present invention in a suitable vegetable oil, for example arachis oil or aqueous solutions or suspensions.

Preferably, a solid composition comprises a) 10-99% active combination; b) 1-90% of a diluent; c) 0.1-10% of a lubricating agent; d) 0.1-15% of a disintegrating agent; and optionally e) 0.1-15% of a binder. Optionally 0.1-10% of a flow aid may be added.

Suitable diluents include lactose, calcium phosphate, dextrin, microcrystalline cellulose, sucrose, starch, modified starch, calcium sulphate or mixtures thereof. Suitable lubricating agents include magnesium stearate, stearic acid, calcium stearate, sodium stearyl fumarate (sold under the trade name PRUV), hydrogenated vegetable oils or mixtures thereof. Preferably the lubricating agent is magnesium stearate or stearic acid. Suitable disintegrating agents include maize starch, sodium starch glycolate, low substituted hydroxypropyl cellulose, alginic acid, calcium carboxymethyl cellulose, croscarmellose sodium or mixtures thereof. Suitable binders includes polyvinyl pyrrolidone, gelatin, hydroxypropylmethyl cellulose, starch or mixtures thereof. Preferably the binder is polyvinylpyrrolidone. Suitable flow aids include talc and colloidal silicon dioxide. It will be appreciated by those skilled in the art that a particular excipient may perform more than one function for example maize starch may act as a diluent, a binder or as a disintegrating agent.

Controlled release forms of the pharmaceutical compositions of the present invention include rapid release formulations such as soluble granules or melt filled fast release capsules, delayed release formulations such as tablets provided with enteric coatings, for example, of cellulose acetate phthalate and, in particular, sustained release formulations. Numerous types of sustained release formulations are known to those skilled in the art. Typically, the active combination may be encapsulated within a release retarding coating, for example, a copolymer of cellulose ether and acrylate, or may be bound to small particles such as, for example, ion exchange resin beads. Alternatively, the active combination may be incorporated into a matrix containing a release retarding agent such as a hydrophilic gum e.g. xanthan gum, a cellulose derivative e.g. hydroxypropyl methylcellulose, or a polysaccharide, wax or plastics material. Such techniques may provide sustained blood levels of ibuprofen and prochlorperazine by controlling, for example, erosion, swelling, disintegration and dissolution of the composition within the gastrointestinal tract.

The active combination may be formulated into a solid dosage form in which the two active drugs are kept separate. For example, the dosage form may be a bilayer tablet in which the active drugs are contained in different layers. The different layers can be formulated so as to provide the optimum release profile for each drug.

Liquid fill compositions for example viscous liquid fills, liquid paste fills or thixotropic liquid fills are also suitable for oral administration. Melt filled compositions may be obtained by mixing the active combination with certain esters of natural vegetable oil fatty acids, for example, the Gelucire (Trademark) range available from Gattefosse to provide a variety of release rates. Suitably a melt-filled capsule comprises a) 10-80% active combination and b) 20-90% of a fatty acid ester excipient which comprises one or more polyol esters and triglycerides of natural vegetable oil fatty acids.

Solid compositions designed to effervesce when added to water to form an effervescent solution or suspension are also suitable for oral administration. Suitably an effervescent composition comprises a) 1-50% of active combination and b) a pharmaceutically acceptable effervescent couple. Such a composition may be presented in the form of tablets or granules. Preferably, effervescent compositions additionally comprise a taste masking component for example a sweetener, a flavouring agent, arginine, sodium carbonate or sodium bicarbonate.

Solid non-effervescent compositions are preferred compositions of the present invention.

Preferably oral liquid compositions comprise a) 0.1-10% active combination possibly as coated particles b) 1-50% of a diluent c) water to 100%. Optionally the composition may contain suspending agents, thickeners, cosolvents such as alcohol and/or preservatives. Suitable diluents include sweetening agents for example sorbitol, xylitol, sucrose, or LYCASIN® (registered trademark of Roquette). Suitable suspending agents or thickeners include cellulose gums, agar or natural gums, for example xanthan gum. Flavourings or other taste-masking agents known to those skilled in the art for example saccharin, sodium saccharin, acesulpham K or aspartame may be added.

Compositions for topical administration may be prepared by dispersing the active combination in a pharmaceutically acceptable cream, ointment or gel. A suitable cream may be prepared by incorporating the active combination in a topical vehicle such as petrolatum and/or light liquid paraffin, dispersed in an aqueous medium using surfactants. A suitable cream comprises a) 1-15% active combination; b) 5-40% of an oily phase; c) 5-15% of an emulsifier; d) 30-85% of water. Suitable oily phases comprise petrolatum and/or light liquid paraffin. Alternatively, the active combination may be distributed in a base comprising a) 10-40% of a self emulsifying base; b) 60-90% of water to form a cream. LABRAFILL and GELOT (both tradenames of Gattefosse) are examples of self emulsifying bases. An ointment may be prepared by mixing the active combination with a topical vehicle such as a mineral oil, petrolatum and/or a wax e.g. paraffin wax or beeswax. A suitable ointment comprises a) 1-15% active combination and b) a topical vehicle to 100%. A gel may be prepared by mixing the active combination with a topical vehicle comprising a gelling agent e.g. basified Carbomer BP, in the presence of water. Suitable gels comprise a) 1-15% active combination; b) 1-20% of a gelling agent; c) 0.01-10% of a preservative and d) water to 100%. Preferably the gelling agent comprises 0.1-10% of a carbomer and a neutralising agent.

Suitable topically administrable compositions may also comprise a matrix in which the active combination is dispersed so that the compounds are held in contact with the skin in order to administer the compounds transdermally, for example in a patch or poultice. A suitable transdermal composition may be prepared by mixing the active combination with a topical vehicle, such as described above, together with a potential transdermal accelerant such as dimethyl sulphoxide, propylene glycol or peppermint oil.

Compositions of the invention suitable for rectal administration are known pharmaceutical forms for such administration, for example suppositories with polyethylene glycol bases or semi-synthetic glycerides. Preferably the composition in the form of a suppository comprises 10-30% active combination and 70-90% of a carrier wherein the carrier is selected from a base which comprises polyethylene glycol or a semi-synthetic glyceride.

Compositions of the invention suitable for parenteral administration are known pharmaceutical forms for such administration, for example sterile suspensions or sterile solutions of the active combination in a suitable solvent.

Spray formulations may be prepared by dissolving or suspending the active combination in a liquid medium that may also contain other ingredients such as stabilising agents, buffering agents, flavourings, sweeteners, colouring agents and preservatives. For example, a spray may be prepared by dissolving water soluble components in water and non-water soluble ingredients in a co-solvent (eg alcohol). The two phases are then mixed and the resulting mixture filtered and placed into dispensing containers. The dispensing containers may be fitted with a metered, manually-operated spray mechanism or the dispenser may contain a pressurised propellant and be fitted with a suitable dispensing valve. The spray and dispensing container may be adapted for nasal administration of the spray.

In some formulations it may be beneficial to use the active combination in the form of particles of very small size, for example as obtained by fluid energy milling.

In the compositions of the present invention the components of the active combination may, if desired, be associated with other compatible pharmacologically active ingredients and/or enhancing agents.

The invention will now be illustrated by the following Examples which are given by way of example only.

In these examples the ingredients are obtained from the sources listed below:-
Microcrystalline cellulose is available from FMC Corporation under the trade name Avicel P.H. 101;
Polyvinylpyrrolidone is available from GAF (GB) Limited under the trade name Plasdone K29-32;
Croscarmellose sodium is available from FMC Corporation under the trade name Ac-Di-Sol;
Colloidal silicon dioxide is available from Degussa under the trade name Aerosil 200; and
Calcium ;carboxymethyl cellulose is available from Carnation Gums, London under the trade name ECG 505.

### Example 1

The example composition comprises the following ingredients:-

| | % w/w |
|---|---|
| Ibuprofen | 57 |
| Prochlorperazine maleate | 0.85 |
| Tricalcium phosphate | 22.15 |
| Microcrystalline cellulose | 6 |
| Polyvinylpyrrolidone (Plasdone) | 3 |
| Croscarmellose sodium | 10 |
| Stearic acid | 1 |

The composition is prepared according to the following stages:-
a) the ibuprofen, prochlorperazine maleate, tricalcium phosphate, croscarmellose sodium and microcrystalline cellulose are sieved and blended to form a homogeneous mixture;
b) the mixture is granulated with a solution of polyvinylpyrrolidone and then dried;
c) the dried granules are blended with the stearic acid;
d) the lubricated granule is compressed to form tablet cores each containing 200mg of ibuprofen and 3mg of prochlorperazine maleate or each containing 400mg of ibuprofen and 6mg of prochlorperazine maleate; and
e) the tablet cores are coated with a film coating comprising the following ingredients:-

| | % w/w |
|---|---|
| Hydroxypropylmethyl cellulose | 65 |
| Titanium dioxide | 20 |
| French chalk | 15 |

### Example 2

In the same manner as described in Example 1 is prepared the composition of Example 2 comprising the following ingredients in the form of tablets each containing 200 mg of ibuprofen and 3 mg of prochlorperazine maleate or each containing 400 mg of ibuprofen and 6 mg of prochlorperazine maleate.

| | % w/w |
|---|---|
| Ibuprofen | 60 |
| Prochlorperazine maleate | 0.9 |
| Tricalcium phosphate | 18.5 |
| Microcrystalline cellulose | 6 |
| Polyvinylpyrrolidone (Plasdone) | 4 |
| Croscarmellose sodium | 10 |
| Stearic acid | 0.6 |

### Example 3

The example composition comprises the following ingredients:

| | %w/w |
|---|---|
| Ibuprofen | 60 |
| Prochlorperazine maleate | 0.9 |
| Tricalcium phosphate | 18.5 |
| Microcrystalline cellulose | 6 |
| Polyvinylpyrrolidone (Plasdone) | 4 |
| Croscarmellose sodium | 10 |
| Magnesium stearate | 0.6 |

The composition is prepared according to the following stages:-
(a) the prochlorperazine maleate and microcrystalline cellulose were triturated;
(b) the ibuprofen, tricalcium phosphate, croscarmellose sodium and triturated mixture from (a) are sieved and blended to form a homogeneous mixture;
(c) the mixture is granulated with a solution of polyvinylpyrrolidone in propan-2-ol and then dried;
(d) the dried granules are blended with the magnesium stearate;
(e) the lubricated granule is compressed to form tablet cores each containing 200 mg of ibuprofen and 3 mg of prochlorperazine maleate or each containing 400 mg of ibuprofen and 6 mg of prochlorperazine maleate; and (e) the tablet cores are coated with a film coating comprising the following ingredients:-

| | % w/w |
|---|---|
| Hydroxypropylmethylcellulose | 65 |
| Titanium dioxide | 20 |
| French chalk | 15 |

### Example 4

In a similar manner to that described in Example 3, compositions containing 200 mg of ibuprofen and 1.5 mg of prochlorperazine or 400 mg of ibuprofen and 3 mg of prochlorperazine maleate are prepared from the following ingredients:-

| | % w/w |
|---|---|
| Ibuprofen | 60.8 |
| Prochlorperazine maleate | 0.46 |
| Microcystalline cellulose | 6 |
| Tricalcium phosphate | 18.14 |
| Croscarmellose sodium | 10 |
| Polyvinylpyrrolidone | 4 |
| Magnesium stearate | 0.6 |

### Example 5

In a similar manner to that described in Example 3, compositions containing 200 mg of ibuprofen and 5 mg of prochlorperazine maleate or 400 mg of ibuprofen and 10 mg of prochlorperazine maleate are prepared from the following ingredients:-

| | % w/w |
|---|---|
| Ibuprofen | 59.5 |
| Prochlorperazine maleate | 1.49 |
| Microcystalline cellulose | 6 |
| Tricalcium phosphate | 18.41 |
| Croscarmellose sodium | 10 |
| Polyvinylpyrrolidone | 4 |
| Magnesium stearate | 0.6 |

### Example 6

The composition of Example 6 comprises the following ingredients:-

| | % w/w |
|---|---|
| Ibuprofen | 56 |
| Prochlorperazine maleate | 1.4 |
| Maize starch | 38.6 |
| Dried maize starch | 3.5 |
| Stearic acid | 0.5 |

The composition is prepared according to the following steps:-
(a) the ibuprofen, prochlorperazine maleate and maize starch are sieved and blended to form a homogeneous mixture;
(b) the mixture is granulated with water and then dried;
(c) the dried granules are blended with the dried maize starch and stearic acid;
(d) the lubricated granule is compressed to form tablet cores each containing 200 mg of ibuprofen and 5 mg of prochlorperazine maleate or each containing 400 mg of ibuprofen and 10 mg of prochlorperazine maleate;
(e) the tablet cores are coated with a sugar coating comprising the following ingredients:-

| | % w/w |
|---|---|
| Opaglos regular | 1.0 |
| Acacia gum | 1.0 |
| Refined sugar | 73.5 |
| Calcium sulphate | 23.5 |
| Sodium carboxymethyl cellulose | 1.0 |

### Example 7

In the same manner as described in Example 3 the composition of Example 7 is prepared comprising the following ingredients in the form of tablets each containing 200 mg of ibuprofen and 5 mg of prochlorperazine maleate or each containing 400 mg of ibuprofen and 10 mg of prochlorperazine maleate:-

| | % w/w |
|---|---|
| Ibuprofen | 59 |
| Prochlorperazine maleate | 1.48 |
| Maize starch | 35.52 |
| Dried maize starch | 3.5 |
| Stearic acid | 0.5 |

### Example 8

The composition of Example 8 comprises the following ingredients:-

| | % w/w |
|---|---|
| Ibuprofen | 70 |
| Prochlorperazine maleate | 1.05 |
| Sodium lauryl sulphate | 0.3 |
| Maize starch | 21.25 |
| Pregelled starch | 6 |
| Colloidal silicon dioxide | 0.4 |
| Magnesium stearate | 0.1 |

The example composition is prepared according to the following stages:-
a) the ibuprofen, prochlorperazine maleate, sodium lauryl sulphate, maize starch, pregelled starch, silicone dioxide and magnesium stearate are blended together to form a homogeneous mixture; and
b) the mixture is filled into hard gelatin capsules, each containing 200 mg of ibuprofen and 3mg of prochlorperazine maleate or each containing 400 mg of ibuprofen and 6 mg of prochlorperazine maleate.

### Example 9

In the same manner as described in Example 8, the composition of Example 9 is prepared comprising the following ingredients to give capsules each containing 200mg of ibuprofen and 1.5 mg prochlorperazine maleate or each containing 400 mg of ibuprofen and 3 mg of prochlorperazine maleate.

| | % w/w |
|---|---|
| Ibuprofen | 75.5 |
| Prochlorperazine maleate | 0.57 |
| Sodium lauryl sulphate | 0.3 |
| Maize starch | 16.23 |
| Pregelled starch | 6 |
| Colloidal silicon dioxide | 0.4 |
| Magnesium stearate | 1 |

### Example 10

The composition of Example 10 is prepared comprising the following ingredients:-

| | % w/w |
|---|---|
| Ibuprofen | 61.54 |
| Prochlorperazine maleate | 0.92 |
| Microcrystalline cellulose | 11.04 |
| Croscarmellose sodium | 10 |
| Stearic acid | 0.5 |
| Pregelled starch | 16 |

The example composition is prepared as follows:
1) The ibuprofen, prochlorperazine maleate, microcystalline cellulose, 5% of the croscarmellose sodium and the pregelled starch are granulated with water.
2) The granules are dried and sized, blended with the other ingredients and compressed into tablets which contain 200mg ibuprofen and 3mg prochlorperazine maleate. The tablets are then film coated.

### Example 11

In a similar manner to that described in Example 10, the composition of Example 11 is prepared from the following ingredients to give tablets each containing 200 mg of ibuprofen and 5 mg of prochlorperazine maleate or each containing 400 mg of ibuprofen and 10 mg of prochlorperazine maleate.

| | % w/w |
|---|---|
| Ibuprofen | 61.54 |
| Prochlorperazine maleate | 1.54 |
| Microcrystalline cellulose | 10.42 |
| Croscarmellose sodium | 16 |
| Stearic acid | 0.5 |
| Pregelled starch | 10.00 |

### Example 12

The composition of Example 12 is prepared from the following ingredients to give tablets each containing 200 mg of ibuprofen and 5 mg of prochlorperazine maleate or each containing 400 mg of ibuprofen and 10mg of prochlorperazine maleate.

| | % w/w |
|---|---|
| Ibuprofen | 57.14 |
| Prochlorperazine maleate | 1.43 |
| Microcrystalline cellulose | 29.68 |
| Polyvinylpyrrolidone | 0.71 |
| Stearic acid | 0.54 |
| Colloidal silicon dioxide | 0.50 |
| Calcium carboxymethyl cellulose | 10.00 |

The composition is prepared in the following manner
1) The ibuprofen, prochlorperazine maleate, microcystalline cellulose, part (20%) of the calcium carboxymethyl cellulose are granulated using a solution of polyvinylpyrrolidone in propan-2-ol.
2) The granules are sized and dried to remove propan-2-ol.
3) The dried granules are blended with the remaining ingredients and compressed into tablets which may be coated if required.

### Example 13

In a similar manner to that described in Example 12 the following ingredients are used to prepare tablets containing 200 mg of ibuprofen and 10 mg of prochlorperazine maleate.

| | %w/w |
|---|---|
| Ibuprofen | 57.14 |
| Prochlorperazine maleate | 2.86 |
| Microcrystalline cellulose | 28.29 |
| Polyvinylpyrrolidone | 0.71 |
| Stearic acid | 0.50 |
| Colloidal silicon dioxide | 0.50 |
| Calcium carboxymethyl cellulose | 10.00 |

### Example 14

A bilayer tablet which contains 200 mg of ibuprofen in a sustained release layer and 10 mg of prochlorperazine maleate in the other layer from which it is released soon after ingestion is prepared from the following ingredients.

| | % w/w |
|---|---|
| Ibuprofen containing layer | |
| Ibuprofen | 77.22 |
| Polyvinylpyrrolidone | 2.51 |
| Stearic acid | 1.00 |
| Xanthan gum | 19 |
| Colloidal silicon dioxide | 0.27 |
| Prochlorperazine maleate containing layer | |
| Prochlorperazine maleate | 2.06 |
| Tricalcium phosphate | 56.78 |
| Microcrystalline cellulose | 20.16 |
| Polyvinylpyrrolidone | 4.5 |
| Croscarmellose sodium | 15.5 |
| Magnesium stearate | 1 |

The method of making the tablet is as follows:
A) Ibuprofen-containing layer
   The ibuprofen, 3% of the xanthan gum are mixed and granulated using polyvinylpyrrolidone in propan-2-ol. The granules are dried, sized and then blended with the remaining ingredients.
B) Prochlorperazine maleate-containing layer
   The prochlorperazine maleate, tricalcium phosphate, microcrystalline cellulose and croscarmellose sodium are mixed and granulated using polyvinylpyrrolidone in aqueous propan-2-ol. The granules dried, sized and blended with the remaining ingredients.
C) Tablet production
   Using a bilayer tabletting press, the prochlorperazine maleate-containing layer is compressed followed by the ibuprofen-containing layer to give bilayer tablets which may be coated if required

### Example 15

The composition of Example 15 is prepared by mixing the ingredients listed below and preparing tablets each containing 200mg of ibuprofen and 3 mg of prochlorperazine maleate or each containing 400 mg of ibuprofen and 6mg of prochlorperazine maleate by direct compression.

| | % w/w |
|---|---|
| Ibuprofen | 78.03 |
| Prochlorperazine maleate | 1.17 |
| Microcrystalline cellulose | 13.5 |
| Stearic acid | 1 |
| Lactose | 5.2 |
| Sodium lauryl sulphate | 1 |
| Colloidal silicon dioxide | 0.1 |

### Example 16

In a similar manner to that described in Example 16, directly compressed tablets each containing 200 mg of ibuprofen and 5 mg of prochlorperazine maleate are prepared from the following ingredients:-

| | % w/w |
|---|---|
| Ibuprofen | 78.03 |
| Prochlorperazine maleate | 1.96 |
| Microcrystalline cellulose | 12.71 |
| Stearic acid | 1 |
| Lactose | 5.2 |
| Sodium lauryl sulphate | 1 |
| Colloidal silicon dioxide | 0.1 |

### Example 17

In a similar manner to that described in Example 16, directly compressed tablets each containing 200 mg of ibuprofen and 5 mg of prochlorperazine maleate are prepared from the following to produce a chewable tablet.

| | %w/w |
|---|---|
| Ibuprofen (Taste masked) | 46.03 |
| Prochlorperazine maleate | 1.05 |
| Manitol | 52.30 |
| Magnesium stearate | 0.62 |

Pharmaceutical compositions of the present invention may also be prepared by replacing the ibuprofen in any one of Examples 1 to 16 by the equivalent amount of an ibuprofen salt (eg the sodium or lysine salt) or the active S(+)-enantiomer or a salt thereof and/or by replacing the prochlorperazine maleate by the equivalent amount of another salt of prochlorperazine maleate (eg the mesylate salt).

### Examples 18 - 23

Individual pharmaceutical compositions for oral administration were prepared in accordance with known formulation procedures as follows:

| Component | Example 18 (mg) | Example 19 (mg) | Example 20 (mg) | Example 21 (mg) | Example 22 (mg) | Example 23 (mg) |
|---|---|---|---|---|---|---|
| lbuprofen lysinate | 342 | 342 | 342 | 342 | 342 | 342 |
| Prochlorperazine maleate | 5 | 5 | 5 | 5 | 5 | 5 |
| Microcrystalline cellulose | 180 | 180 | 100 | - | 30 | 80 |
| Sodium starch glycolate | 40 | - | - | - | - | - |
| Silicon dioxide | 1.5 | - | - | - | - | 1.5 |
| Stearic acid | 5 | - | 9 | - | - | 5 |
| Lactose | - | 100 | - | 150 | - | - |
| Croscarmellose sodium | - | 40 | 40 | 40 | 20 | 25 |
| Magnesium stearate | - | 3 | - | 3 | 3 | - |
| Pregelled maize starch | - | - | - | - | - | 35 |
| Tricalcium phosphate | - | - | 50 | - | 60 | - |
| Polyvinylpyrrolidone (plasdone) | - | - | - | - | 12 | - |
| Starch 1500 | - | - | - | - | - | 20 |

## Claims

1. A pharmaceutical composition for the treatment of migraine or overindulgence comprising a therapeutically effective amount of racemic ibuprofen or a pharmaceutically acceptable salt or an enantiomer or a pharmaceutically acceptable salt thereof and a therapeutically effective amount of prochlorperazine or a pharmaceutically acceptable salt thereof.

2. A composition according to claim 1 which comprises a pharmaceutically acceptable diluent or carrier.

3. A composition according to claim 1 or 2, comprising 25 to 400 mg of racemic ibuprofen or the corresponding amount of a pharmaceutically acceptable salt or an enantiomer or a pharmaceutically acceptable salt thereof.

4. A composition according to any one of the preceding claims, comprising 25 to 200 mg of racemic ibuprofen or the corresponding amount of a pharmaceutically acceptable salt or enantiomer or a pharmaceutically acceptable salt thereof.

5. A composition according to any one of the preceding claims, comprising and 0.5 to 5 mg of prochlorperazine or the corresponding amount of a pharmaceutically acceptable salt thereof.

6. A composition according to any one of the preceding claims, comprising 1 to 2.5 mg of prochlorperazine or the corresponding amount of a pharmaceutically acceptable salt thereof.

7. A composition according to any one of the preceding claims, wherein the ibuprofen is present in the form of racemic ibuprofen lysine salt.

8. The use of a combination of a therapeutically effective amount of racemic ibuprofen or a pharmaceutically acceptable salt or an enantiomer or a pharmaceutically acceptable salt thereof and a therapeutically effective amount of prochlorperazine or a pharmaceutically acceptable salt thereof in the preparation of a medicament for the treatment of migraine or overindulgence.

9. Use according to claim 8 for the preparation of a medicament in the form of a unit dose.

10. Use according to claim 9, wherein each unit dose comprises 25 to 400 mg of racemic ibuprofen or the corresponding amount of a pharmaceutically acceptable salt or enantiomer or pharmaceutically acceptable salt thereof and 0.5 to 5 mg of prochlorperazine or the corresponding amount of a pharmaceutically acceptable salt thereof.

11. Use according to claim 10, wherein each unit dose of the medicament comprises 25 to 200 mg of ibuprofen or the corresponding amount of a pharmaceutically acceptable salt or enantiomer or pharmaceutically acceptable salt thereof and 1 to 2.5 mg of prochlorperazine or the corresponding amount of a pharmaceutically acceptable salt thereof.

12. Use according to any one of claims 8 to 11, wherein prochlorperazine maleate is used.

13. Use according to any one of Claims 8 to 12, wherein racemic ibuprofen lysine salt is used.
